# EUROPEAN PATENT APPLICATION

(11) **EP 4 260 905 A1**
(43) Date of publication of application: **18.10.2023**
(21) Application number: 21903423.8
(22) Date of filing: 08.12.2021
(51) Int. Cl.: A61P 1/04, A61K 35/741, A23L 33/135

(54) **COMPOSITION CONTAINING ACIDIPROPIONIBACTERIUM SPP. OR TREATED PRODUCT THEREOF**

(30) Priority: 09.12.2020 JP 2020204427
(71) Applicant: MEIJI CO., LTD, Chuo-ku Tokyo 104-8306 (JP)
(72) Inventor: FUJIWARA Shin, Hachioji-shi, Tokyo 192-0919 (JP); YOKOO Takehiro, Hachioji-shi, Tokyo 192-0919 (JP); TAKEDA Mariko, Hachioji-shi, Tokyo 192-0919 (JP)
(74) Representative: TBK
(86) International application number: PCT/JP2021/045043
(87) International publication number: WO 2022/124324

(57) **Abstract**

Provided is a novel means for promoting proliferation of a bacterium of the genus *Faecalibacterium,* that is, a composition containing a bacterium of the genus *Acidipropionibacterium* or a processed product thereof and satisfying at least one requirement selected from the following (A) to (D):
(A) the composition is a composition for promoting proliferation of a bacterium of the genus *Faecalibacterium*;
(B) the composition is a composition for improving symptoms or conditions involving a bacterium of the genus *Faecalibacterium*;
(C) the composition is an alternative to a saccharide that promotes proliferation of a bacterium of the genus *Faecalibacterium*;
(D) a total content of a saccharide that promotes proliferation of a bacterium of the genus *Faecalibacterium* in the composition is 15 mass% or less.

## Description

### Cross Reference to Related Application

This patent application claims the priority of Japanese Patent Application No. 2020-204427 filed December 9, 2020, the whole contents of which are incorporated herein by reference.

### Technical Field

The present invention relates to a composition comprising a bacterium of the genus *Acidipropionibacterium* or a processed product thereof. More specifically, the present invention relates to a composition comprising a bacterium of the genus *Acidipropionibacterium* or a processed product thereof and to be used in promoting proliferation of a bacterium of the genus *Faecalibacterium.*

### Background Art

*Faecalibacterium prausnitzii* (hereinafter referred also to "*F*. *prausnitzii*") is known as a typical bacterial strain of the genus *Faecalibacterium* and a dominant bacterial species occupying about 5% of the whole intestinal bacteria of a healthy adult, and has recently attracted attention as a bacterial species having a physiological function beneficial to human health, such as butyrate production and anti-inflammatory activity.

It has been found that the relative abundance of F. *prausnitzii* is low in the intestine of patients with irritable bowel syndrome (IBS) or inflammatory bowel disease (IBD), compared to that of healthy persons. Accordingly, it is considered that diseases can be prevented or improved by increasing the bacterial count of *F*. *prausnitzii* in the intestine.

Saccharides such as 1-kestose and inulin, which are less digestible or poorly adsorbed in the small intestine, are reported to increase the bacterial count of *F. prausnitzii* in the intestine (see, for example, Patent Documents 1 and 2). However, it is reported that the saccharides (such as 1-kestose and inulin) have a risk of worsening symptoms of irritable bowel syndrome (IBS), such as abdominal bloating (see, for example, Non-Patent Document 1).

The saccharides such as 1-kestose and inulin, which are less digestible or poorly adsorbed in the small intestine, are also referred to as FODMAP or FOLFAP. FODMAP is the acronym of Fermentable Oligosaccharides, Disaccharides, Monosaccharides and Polyols. Another term substantially equal to FODMAP and commonly used is FOLFAP, which is the acronym of Fructose, Oligosaccharide, Lactose, Fructans and Polyols.

FODMAP and FOLFAP refer to a group of carbohydrates that are obtained from molecules found in foods but not able to be sufficiently absorbed by some people. When these molecules are not sufficiently absorbed in the small intestine of the gastrointestinal tract, they proceed to the large intestine through the gastrointestinal tract and usually serve as food sources for resident bacteria in the large intestine. The resident bacteria digest/ferment FODMAP and FOLFAP. The activity of these bacteria sometimes causes symptoms of irritable bowel syndrome (IBS).

Accordingly, it has been desired to develop a novel means that safely promotes proliferation of a bacterium of the genus *Faecalibacterium* in living bodies, as an alternative to FODMAP or FOLFAP.

### Citation List

### Patent Documents

Patent Document 1: WO 2017-159647
Patent Document 2: National Publication of International Patent Application No. 2015-535280

### Non-Patent Document

Non-Patent Document 1: Hayes PA et al., "Irritable bowel syndrome: the role of food in pathogenesis and management", 2014

### Summary of Invention

The present inventors have conducted intensive studies on the problem. As a result, they have found that proliferation of a bacterium of the genus *Faecalibacterium* can be promoted by a bacterium of the genus *Acidipropionibacterium* or a processed product thereof. The present invention is based on the finding.

An object of the present invention is to provide a novel means for promoting proliferation of a bacterium of the genus *Faecalibacterium.*

According to an embodiment of the present invention, there is provided a composition comprising a bacterium of the genus *Acidipropionibacterium* or a processed product thereof and satisfying at least one requirement of the following (A) to (D):
(A) the composition is a composition for promoting proliferation of a bacterium of the genus *Faecalibacterium;*
(B) the composition is a composition for improving symptoms or conditions involving a bacterium of the genus *Faecalibacterium;*
(C) the composition is an alternative to a saccharide that promotes proliferation of a bacterium of the genus *Faecalibacterium;* and
(D) the content of a saccharide that promotes proliferation of a bacterium of the genus *Faecalibacterium* in the composition is 15 mass% or less.

According to the present invention, it is possible to promote proliferation of a bacterium of the genus *Faecalibacterium* by using a bacterium of the genus *Acidipropionibacterium* or a processed product thereof. Almost all bacterial strains of the genus *Acidipropionibacterium* can be isolated from raw milk collected in various places in Japan. Since *P. acidipropionici* (old name of the species) is included in the QPS (Qualified Presumption of Safety) list of microorganisms having qualified presumption of safety issued by the EFSA (European Food Safety Authority), the present invention is advantageous in promoting proliferation of a bacterium of the genus *Faecalibacterium* while ensuring biosafety. In the present invention, a bacterium of the genus *Acidipropionibacterium* can be used as an alternative to FODMAP and FOLFAP and is advantageous in promoting proliferation of a bacterium of the genus *Faecalibacterium* in an environment where FODMAP and FOLFAP are present in small amounts. The present invention can be advantageously used for improving diseases and disease conditions involving a bacterium of the genus *Faecalibacterium.*

### Brief Description of the Drawings

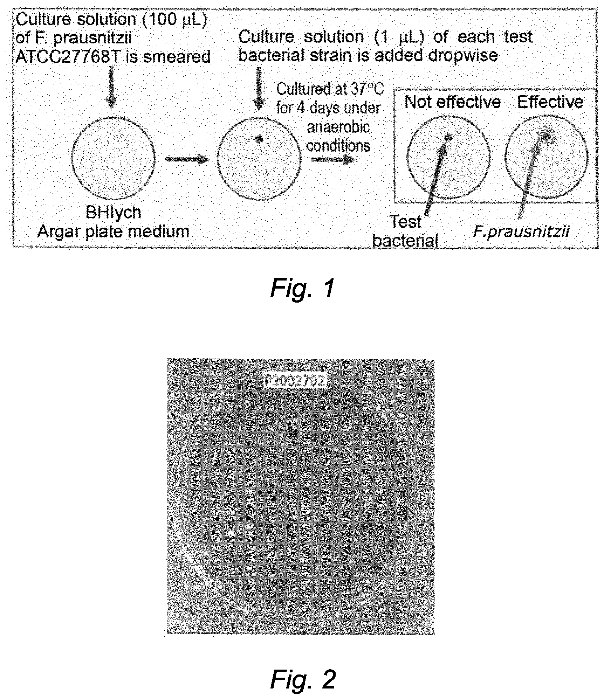

Figure 1 shows a scheme of an experiment (Example 1) for confirming the presence or absence of a proliferation promoting effect of a test bacterial strain (*F. prausnitzii* ATCC27768T) by adding a test sample dropwise in an agar medium containing the test bacterial strain and subjecting the medium to stationary culture;
Figure 2 is a photograph of an agar medium (Example 1) after stationary culture which was performed by adding A. *acidipropionici* P2002702 dropwise to the agar medium containing a test bacterial strain (*F. prausnitzii* ATCC27768T);

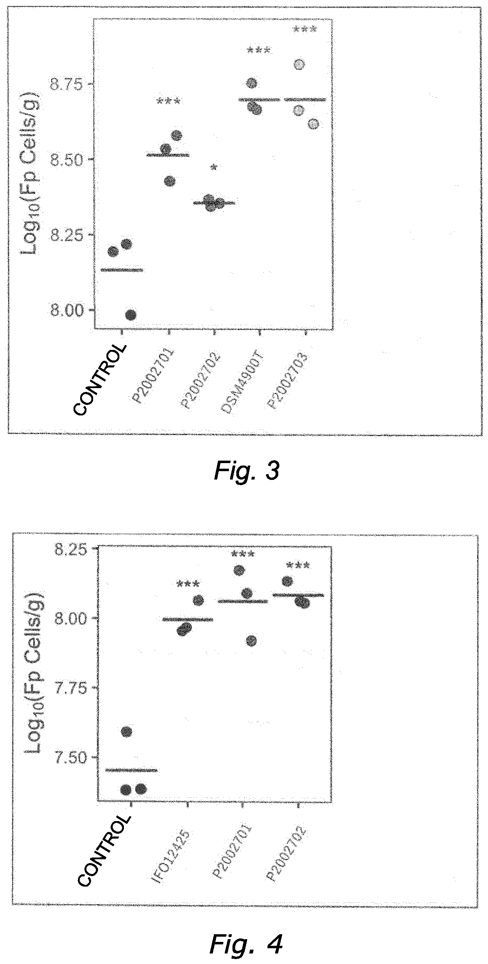

Figure 3 is a graph showing the bacterial counts of a test bacterial strain after a test sample (*A. acidipropion* P2002701, P2002702, DSM4900T or P2002703) or a control (saline) was added to a culture solution (liquid medium) containing the test bacterial strain (*F. prausnitzii* ATCC27768T) and co-cultured; and
Figure 4 is a graph showing the bacterial counts of a test bacterial strain after a test sample (culture supernatant of *A. acidipropionici* P2002701, P2002702 or IFO12425) or a control (saline) was added to a culture solution (liquid medium) containing the test bacterial strain (*F. prausnitzii* ATCC27768T) and co-cultured.

### Detailed Description of the Invention

According to an embodiment of the present invention, there is provided a composition comprising a bacterium of the genus *Acidipropionibacterium* or a processed product thereof and satisfying at least one requirement selected from the following (A) to (D):
(A) the composition is a composition for promoting proliferation of a bacterium of the genus *Faecalibacterium*;
(B) the composition is a composition for improving symptoms or conditions involving a bacterium of the genus *Faecalibacterium*;
(C) the composition is an alternative to a saccharide that promotes proliferation of a bacterium of the genus *Faecalibacterium*; and
(D) the content of a saccharide that promotes proliferation of a bacterium of the genus *Faecalibacterium* in the composition is 15 mass% or less.

The fact that proliferation of a bacterium of the genus *Faecalibacterium* is effectively promoted by a bacterium of the genus *Acidipropionibacterium* or a processed product thereof is beyond the expectation of those skilled in the art.

It is known that a bacterium of the genus *Acidipropionibacterium* is a gram positive and anaerobic bacillus.

Examples of bacterial species of the *Acidipropionibacterium* include *Acidipropionibacterium acidipropionici* (e.g., IFO12425 strain, P2002701 strain, P2002702 strain, DSM4900T strain, P2002703 strain), *Acidipropionibacterium damnosum, Acidipropionibacterium jensenii, Acidipropionibacterium microaerophilum, Acidipropionibacterium olivae* and *Acidipropionibacterium thoenii. Acidipropionibacterium acidipropionici* is a preferable example.

*Bifidobacterium longum* JCM1217T strain is available from the RIKEN BioResource Research Center Microbe Division (3-1-1 Koyadai, Tsukuba, Ibaraki, Japan) based on the JCM number.

*Acidipropionibacterium acidipropionici* P2002701 strain, P2002702 strain or P2002703 strain is stored by Meiji Co., Ltd. (Meiji Innovation Center, 1-29-1 Nanakuni, Hachioji City, Tokyo, postal code: 192-0919, Japan).

*Acidipropionibacterium acidipropionici* DSM4900T strain is available from the German Collection of Microorganisms and Cell Cultures GmbH based on the DSM number.

*Acidipropionibacterium acidipropionici* IFO12425 strain is available from the National Institute of Technology and Evaluation based on the IFO number.

A bacterium of the genus *Acidipropionibacterium* may be viable or dead bacterium but viable bacterium is preferable.

A bacterium of the genus *Acidipropionibacterium* may be physicochemically processed and used for carrying out the present invention. Examples of a processed product of a bacterium of the genus *Acidipropionibacterium* include a suspension, a culture, culture supernatant, a fermented product, a heat-processed product (e.g., a dissolved material), a ground material, a concentrate, a sterilized product, a liquefied product (e.g., extract), a paste, a dried product (for example, spray dried product, vacuum dried product, drum-dried product, a lyophilizate or lyophilized/ground material such as lyophilized powder) and a diluted product. A culture or culture supernatant is a preferable example. The above processed product may be separated, for example, by any one of chromatographic methods and put in use.

Culturing of a bacterium of the genus *Acidipropionibacterium* is not particularly limited and the bacterium can be cultured in a medium commonly used for microbial culture. More specifically, any culture medium can be used as long as it contains a carbon source (e.g., lactose, glucose, sucrose, fructose, galactose, blackstrap molasses), a nitrogen source (organic nitrogen-containing substance such as a casein hydrolysate, a whey protein hydrolysate and a soy protein hydrolysate), and other nutrients such as inorganic substances.

The culture medium can be prepared by blending nutrient sources, such as a soluble starch, whey, casein, a skimmed milk powder, a whey protein concentrate (WPC), a whey protein isolate (WPI), a yeast extract, a meat extract, a liver extract, a soybean extract, peptone such as Trypticase, protease peptone, a digested serum powder, saccharides such as glucose and lactose, a reducing agent such as L-cysteine hydrochloride and sodium thioglycolate, potassium dihydrogen phosphate, sodium chloride and a mineral such as a whey mineral; or a food containing them in a large amount or a product obtained by treating the food with an enzyme. Although it is not limited, it is preferable that whey or an enzymatic hydrolysate thereof is added to a culture medium.

Culture can be anaerobically or aerobically carried out. Anaerobic or aerobic culture using a liquid medium is preferable. The time for culturing is not particularly limited and usually 0.5 to 200 hours, preferably 5 to 100 hours, more preferably 6 to 80 hours, further preferably 8 to 40 hours and still further preferably 10 to 24 hours. The temperature for culturing is not particularly limited, usually 20 to 50°C, preferably 25 to 45°C, and more preferably 30 to 40°C. Culture can be anaerobically carried out while supplying carbon dioxide gas or nitrogen gas, or aerobically carried out by stirring or shaking while supplying air. Culture is preferably carried out, for example, by inoculating a bacterium of the genus *Acidipropionibacterium* in a sterilized culture medium and aerobically incubating it usually at 30 to 40°C (more preferably 30°C) for 24 to 144 hours in a nitrogen atmosphere.

Bacterial cells of a bacterium of the genus *Acidipropionibacterium* may be isolated from a culture solution by, e.g., filtering or centrifugating a culture and put in use. The bacterial cells isolated can be directly used or may be subjected to a process such as a concentration process, a griding process, a pasting-formation process, a sterilization process, a heating process (e.g., dissolution treatment), a dilution process or a drying process. Such a processed product of the isolated bacterial cells, that is, a concentrated product, a ground product, a past-like product, a sterilized product, a diluted product or a dried product, can be also used.

A culture solution can be directly used as a processed product of a bacterium of the genus *Acidipropionibacterium* without being subjected to an isolation process. The culture solution may be subjected to a process such as a concentration process, a pasting-formation process, a sterilization process, a heating process (e.g., dissolution treatment), a dilution process or a drying process. A processed product of a culture solution such as a concentrated product, paste like product, a sterilized product, a diluted product or a dried product can be used.

A method for preparing culture supernatant is not particularly limited. The culture supernatant is prepared, for example, by centrifuging a culture solution, collecting the supernatant and filtering it to remove bacterial cells. The culture supernatant may be subjected to a process such as a concentration process, a pasting-formation process, a sterilization process, a heating process (e.g., dissolution treatment), a dilution process or a drying process. A processed product of a culture supernatant such as a concentrated product, a paste-like product, a sterilized product, a diluted product or a dried product can be used.

According to an embodiment, the lower limit of the content of a bacterium of the genus *Acidipropionibacterium* or a processed product thereof in a composition of the present invention is, for example, 0.01 mass% or more, preferably 0.1 mass% or more, more preferably 1 mass% or more, further preferably 5 mass% or more, and still further preferably 10 mass% or more. The upper limit thereof is, for example, less than 100 mass%, preferably 95 mass% or less, more preferably 90 mass% or less, further preferably 80 mass% or less and still further preferably 50 mass% or less or 40 mass% or less. Accordingly, the content of a bacterium of the genus *Acidipropionibacterium* or a processed product thereof in a composition of the present invention may fall within the range of, for example, 0.01 mass% or more and less than 100 mass%. In order to effectively promote proliferation of a bacterium of the genus *Faecalibacterium,* the content is preferably 0.1 to 95 mass%, more preferably 1 to 95 mass%, further preferably 5 to 90 mass%, and still further preferably 10 to 80 mass%, 10 to 50 mass% or 10 to 40 mass%.

A bacterium of the genus *Faecalibacterium,* which is a target of the present invention for promoting proliferation, is an intestinal bacterium. A bacterium of the genus *Faecalibacterium* is known as a butyric acid-producing bacterium (good bacteria), which produces short chain fatty acids, particularly, butyric acid, in a large amount, in the large intestine.

As a bacterial species of the genus *Faecalibacterium,* for example, *Faecalibacterium prausnitzii* is preferable.

According to a preferable embodiment, a composition of the present invention may contain additives other than a bacterium of the genus *Acidipropionibacterium* or a processed product thereof. Examples of the additives include, medium components, components acceptable for oral intake (carbohydrates (saccharides and dietary fibers), proteins, lipids, vitamins, minerals, bio-essential trace metals, carriers acceptable from a food sanitation or pharmaceutical point of view).

Examples of medium components include additives for culture such as additives for cellular culture and additives for microbial culture. Examples of the additives for culture include a yeast extract, a meat extract, glucose, an encephalomyo infusion, peptone, protease peptone, a protein hydrolysate (e.g., soybean hydrolysate, gelatin hydrolysate), hemin, amino acids, a digested serum powder, a liver extract, a soluble starch, L-cysteine hydrochloride and sodium thioglycolate. A protein hydrolysate, a yeast extract, a meat extract or glucose is preferable.

Examples of the carbohydrates include saccharides such as a monosaccharide, a disaccharide, a fermentable oligosaccharide; modified starches such as dextrin, a soluble starch, British starch, an oxidized starch, a starch ester and a starch ether; polyols such as xylitol and sorbitol; and dietary fibers.

Examples of the proteins include animal/vegetable proteins such as a whole milk powder, a skimmed milk powder, a partially skimmed milk powder, casein, a whey powder, a whey protein, a whey protein concentrate, a whey protein isolate, α-casein, β-casein, κ-casein, β-lactoglobulin, α-lactalbumin, lactoferrin, soy protein, chicken egg protein, meat protein, and hydrolysates of these; and milk-derived components such as butter, milk minerals, cream, whey, non-protein nitrogen, sialic acid, phospholipid and lactose.

Examples of the lipids include animal fats and oils such as lard, fish oils, and fractionated oils and hydrogenated oils and transesterified oils of these; and vegetable fats and oils such as palm oil, safflower oil, corn oil, rapeseed oil, coconut oil, and fractionated oils, hydrogenated oils and transesterified oils of these.

Examples of the vitamins include vitamin A, carotenes, vitamin B family, vitamin C, vitamin D family, vitamin E, vitamin K family, vitamin P, vitamin Q, niacin, nicotinic acid, pantothenic acid, biotin, inositol, colin and folic acid.

Examples of the minerals include calcium, potassium, magnesium, sodium, copper, iron, manganese, zinc and selenium. To be more specific, potassium dihydrogen phosphate and sodium chloride are included.

Examples of the bio-essential trace metals include manganese sulfate, zinc sulfate, magnesium chloride and potassium carbonate.

Examples of the carriers acceptable from a food sanitation or pharmaceutical point of view include a solvent, an excipient, a stabilizer, a preservative, a moisturizer, an emulsifier, a lubricant, a sweetener, a coloring agent, a flavor, a buffer, an antioxidant, a surfactant and a pH adjuster. More specifically, examples of the carriers include water, an organic solvent, collagen, a polyvinyl alcohol, a polyvinylpyrrolidone, a carboxyvinyl polymer, sodium alginate, a water-soluble dextran, a water-soluble dextrin, sodium carboxymethyl starch, pectin, xanthan gum, gum Arabic, casein, gelatin, agar, propylene glycol, polyethylene glycol, vaseline, paraffin, stearyl alcohol, stearic acid, human serum albumin, and artificial cellular structure such as a liposome.

A composition of the present invention can be produced, for example, by mixing a bacterium of the genus *Acidipropionibacterium* or a processed product thereof and the aforementioned additives in accordance with a process known to those skilled in the art.

Since the bacterium of the genus *Acidipropionibacterium* or a processed product thereof has a proliferation promoting effect on a bacterium of the genus *Faecalibacterium,* it can be used as an alternative to saccharides, such as FODMAP and FOLFAP, that promote proliferation of a bacterium of the genus *Faecalibacterium.*

The bacterium of the genus *Acidipropionibacterium* or a processed product thereof is preferably used for reducing the amount of saccharides such as FODMAP and FOLFAP for use in promoting proliferation of a bacterium of the genus *Faecalibacterium.* The lower limit of the total content of a saccharide that promotes proliferation of a bacterium of the genus *Faecalibacterium* in a composition of the present invention is, for example, 0.01 mass% or more, preferably 0.1 mass% or more, and more preferably 0.5 mass% or more. The upper limit thereof is, for example, 15 mass% or less, preferably 10 mass% or less, more preferably 5 mass% or less, and further preferably 0.8 mass% or less. Accordingly, the total content of a saccharide that promotes proliferation of a bacterium of the genus *Faecalibacterium* in a composition of the present invention is, for example, 15 mass% or less, preferably 0.01 to 10 mass%, more preferably 0.1 to 5 mass%, and further preferably 0.5 to 0.8 mass%.

The saccharide that promotes proliferation of a bacterium of the genus *Faecalibacterium* is, for example, at least one selected from the group consisting of a fermentable carbohydrate, a fermentable oligosaccharide, a monosaccharide, a disaccharide and a polyol. At least one of 1-kestose and inulin is preferable.

The contents of FODMAP and FOLFAP that promote proliferation of a bacterium of the genus *Faecalibacterium* can be measured in accordance with the method described in J. R. Biesiekierski et al. "Quantification of fructans, galacto-oligosacharides and other short-chain carbohydrates in processed grains and cereals", Journal of Human Nutrition and Dietetics, Vol. 24, (2): pp154-176, (April, 2011).

A composition of the present invention can be prepared by using a bacterium of the genus *Acidipropionibacterium* that can be isolated from raw milk collected in various places in Japan or a processed product thereof, as an active ingredient. Because of this, the composition of the present invention can be safely taken by mouth. Accordingly, a composition of the present invention according to an embodiment is an oral composition. According to a preferable embodiment, a composition of the present invention is a food composition or a pharmaceutical composition.

If a composition of the present invention is a food composition, the form of the composition is not particularly limited. Examples of the form include a solution, a suspension, an emulsion, a powder, a paste, a semi-solid molding and a solid molding. Examples of foods and drinks include milk beverages, soft drinks, fermented beverages, fermented milk, lactobacillus beverages, lactic drinks, yogurts, cheeses, butters, creams, ice creams, frozen desserts, chocolates, tablets, gummies, breads, bars, supplements, liquid diets, diets for patients, nutritional foods, frozen foods, processed foods, seasonings and other commercially available foods.

As described above, in a composition of the present invention, the amount of FODMAP and FOLFAP taken by mouth can be reduced by use of a bacterium of the genus *Acidipropionibacterium* or a processed product thereof. Accordingly, a composition of the present invention according to an embodiment is provided as a low-FODMAP diet or a low-FOLFAP diet.

A low-FODMAP diet is described and defined in Gearry R. B et al., JCC, 2008, 09, 004, p8 to 14. More specifically, a low-FODMAP diet contains glucose/100 g plus less than 0.15 g of fructose; or less than 3 g of fructose per diet, and less than 0.2 g of fructan per diet (excluding less than 0.3 g per diet of grains, nuts, and seeds) regardless of the content of glucose.

Accordingly, when a composition of the present invention is provided as a low-FODMAP diet or a low-FOLFAP diet, it is preferable to formulate a preparation so as to contain 0.5 g or less, 0.4 g or less or 0.3 g or less of FODMAPs per diet.

If a composition of the present invention is a pharmaceutical composition, the form of the composition is not particularly limited. If the pharmaceutical composition is an oral preparation, the oral preparation may be in the form of a solid preparation such as a tablet, a powder, a fine granule, a granule, a capsule, a pill and a sustained release agent; and a liquid preparation such as a solution, a suspension and an emulsion. A pharmaceutical composition is preferably used as an oral preparation as described above but can take a parenteral preparation such as an injection and a suppository.

The composition of the present invention is preferably provided so as to take it by mouth in an amount per day or time as a unit. The amount of the composition taken by mouth per unit is preferably singly packaged.

The frequency of intake of a composition of the present invention per day can be appropriately determined by those skilled in the art depending on the type, age, gender and symptom of a subject as long as the effect of the present invention is not hindered. A composition of the present invention may be given to a subject once or a plurality of times. A composition of the present invention can be taken at a frequency of, for example, 1 to 5 times/day, preferably 1 to 3 times/day and more preferably once a day.

Since a composition of the present invention can promote proliferation of a bacterium of the genus *Faecalibacterium* present in the intestine, the composition is preferably provided as a probiotic, a prebiotic or a synbiotic. The "probiotic" herein refers to live microorganisms useful for improving balance of bacterial flora in the intestine and providing health benefits on the host. The "prebiotic" refers to a substance acting on microorganisms already living in the intestine to provide beneficial effects on the host. The "synbiotic" refers to a combination of a probiotic and a prebiotic.

A composition of the present invention containing a bacterium of the genus *Acidipropionibacterium* or a processed product thereof as an active ingredient can be used for improving symptoms or conditions involving a bacterium of the genus *Faecalibacterium.* The "improving" diseases or conditions herein includes treating and preventing diseases or conditions. The "treating" diseases or conditions refers to stopping, lessening or delaying progression or exacerbation of diseases or conditions by not only a medical action but also a non-medical action. The "preventing" diseases or conditions refers to preventing occurrence or recurrence of diseases before it happens by a medical action or a non-medical action in preparation for conceivable worsening of them.

Note that, in the specification, symptoms and diseases are not distinguishable and interchangeably used.

Examples of the diseases or conditions involving a bacterium of the genus *Faecalibacterium* include irritable bowel syndrome (IBS), inflammatory bowel disease (IBD), glycometabolism, lipid metabolism, intestinal environment, immune status, oxidative stress, inflammation, colorectal cancer and depression. Irritable bowel syndrome (IBS) and inflammatory bowel disease (IBD) are preferable examples. Herein, examples of irritable bowel syndrome (IBS) include bloating and dilatation, excessive production of gas (flatulence), abdominal pain, nausea, alteration in bowel habit (diarrhea, constipation or both) and other gastrointestinal symptoms, caused by IBS.

A composition of the present invention can be provided as food or pharmaceuticals with claims of uses (as mentioned above). Claims of uses are preferably claims permitted by, e. g., public administrations (for example, claims made in accordance with approvals by public administrations based on various administrative systems). Examples of claims include claims as health foods, dietary supplements, functional foods, health preservation foods (for example, foods for specified health use, foods with nutrient function, foods with a function claim) and claims as a food with a special purpose (for example, infant foods, foods for pregnant women, foods for the elderly, patient foods). Other than these, claims approved by the Health, Labour and Welfare Ministry, such as claims for foods for specified health uses and claims approved by analogous systems (regulations) are included in the examples. Examples of claims of the latter case include claims as foods for specified health uses, claims as foods for conditionally specified health uses, claims of affecting the structure or function of the body and claims of reduction of disease risk. More specifically, examples of claims include claims as foods for specified health uses (particularly uses for health purpose) defined in the ordinance for enforcement of the Health Promotion Act (by ordinance No. 86 of the Ministry of Health, Labour and Welfare in Japan issued as of April 30, 2003) and analogous claims thereto.

Specific expressions of claims are as follows: "probiotics", "prebiotics", "synbiotics", "persons who want to increase *Faecalibacterium*", "regulation of the functions of the intestines", "for improvement of microbiota ", "for improvement of irritable bowel syndrome (IBS)", "for improvement of inflammatory bowel disease (IBD)", "for improvement abdominal symptoms", "low-FODMAP" and "low-FOLFAP". Claims derived from the effects of the present invention are all included. These claims may be attached to, e.g., packages and containers, and described in, e.g., catalogs, pamphlets and web pages.

According to another embodiment of the present invention, there is provided a method for promoting proliferation of a bacterium of the genus *Faecalibacterium,* including a step of allowing a subject in need to take an effective amount of a bacterium of the genus *Acidipropionibacterium* or a processed product thereof. The subject is preferably a subject who needs a low-FODMAP diet or a low-FOLFAP food. According to another preferable embodiment, the above method is provided as a method for improving symptoms or conditions involving a bacterium of the genus *Faecalibacterium.*

According to an embodiment, medical actions to humans are excluded from the range of the method of the present invention. The medical actions to humans refer to actions administering pharmaceuticals under prescription by doctors.

The term "effective amount" in the method of the present invention is preferably an amount effective for promoting proliferation of a bacterium of the genus *Faecalibacterium.* According to an embodiment, the effective amount in the method of the present invention can be determined as the amount of a bacterium of the genus *Acidipropionibacterium* or a processed product thereof to be taken per day. According to a preferable embodiment of the present invention, the lower limit of the effective amount of a bacterium of the genus *Acidipropionibacterium* or a processed product thereof is preferably 0.01 mg/body weight kg/day or more, more preferably 0.1 mg/body weight kg/day or more, further preferably 1 mg/body weight kg/day or more, and still further preferably 5 mg/body weight kg/day or more, 10 mg/body weight kg/day or more, 30 mg/body weight kg/day or more or 80 mg/body weight kg/day or more. The upper limit thereof is preferably 10000 mg/body weight kg/day or less, more preferably 9000 mg/body weight kg/day or less, further preferably 6000 mg/body weight kg/day or less, and still further preferably 5000 mg/body weight kg/day or less or 4000 mg/body weight kg/day or less. Accordingly, according to a preferable embodiment of the present invention, the effective amount of a bacterium of the genus *Acidipropionibacterium* or a processed product thereof is preferably 0.01 to 10000 mg/body weight kg/day, more preferably 0.1 to 9000 mg/body weight kg/day, further preferably 1 to 6000 mg/body weight kg/day, and still further preferably 5 to 6000 mg/body weight kg/day, 10 to 5000 mg/body weight kg/day, 30 to 5000 mg/body weight kg/day or 80 to 4000 mg/body weight kg/day. According to a more preferable embodiment, the effective amount may be determined as the same as the content of a bacterium of the genus *Acidipropionibacterium* or a processed product thereof in a composition of the present invention. The effective amount is usually expressed as an amount in a dry-weight basis.

Although feeding of the present invention is not particularly limited, feeding is preferably tube feeding (e.g., nasal route, gastric fistula) or oral intake, and more preferably oral intake.

The subject to which the present invention is to be applied is not particularly limited as long as the effect of the present invention is exhibited. Examples of the subject include mammals such as a human, a monkey, a chimpanzee, a cow, a horse, sheep and a goat. A human is preferable subject. In the above embodiment, the subject is preferably a healthy subject. As described above, a bacterium of the genus *Acidipropionibacterium* or a processed product thereof can be used for promoting proliferation of a bacterium of the genus *Faecalibacterium* while reducing the amount of FODMAP or FOLFAP to be used. Thus, according to an embodiment, the subject to which the present invention is applied is a subject in need of a low-FODMAP diet or a low-FOLFAP diet.

According to a preferable embodiment, in the method of the present invention, it is preferable that a bacterium of the genus *Acidipropionibacterium* or a processed product thereof are taken simultaneously or sequentially with or a low-FODMAP diet or a low-FOLFAP diet.

According to another preferable embodiment of the present invention, there is provided use of a bacterium of the genus *Acidipropionibacterium* or a processed product thereof in production of a composition satisfying at least one requirement selected from the following (A) to (D):
(A) the composition is a composition for promoting proliferation of a bacterium of the genus *Faecalibacterium;*
(B) the composition is a composition for improving symptoms or conditions involving a bacterium of the genus *Faecalibacterium;*
(C) the composition is an alternative to a saccharide that promotes proliferation of a bacterium of the genus *Faecalibacterium;*
(D) a total content of a saccharide that promotes proliferation of a bacterium of the genus *Faecalibacterium* in the composition is 15 mass% or less.

According to another preferable embodiment of the present invention, there is provided use of a bacterium of the genus *Acidipropionibacterium* or a processed product thereof as at least one of the following (A') to (C'):
(A') a promotor for proliferation of a bacterium of the genus *Faecalibacterium;*
(B') an improver for symptoms or conditions involving a bacterium of the genus *Faecalibacterium*; and
(C') an alternative to a saccharide that promotes proliferation of a bacterium of the genus *Faecalibacterium.*

According to another preferable embodiment of the present invention, there is provided a bacterium of the genus *Acidipropionibacterium* or a processed product thereof for use as at least one of the following (A') to (C'):
(A') a promotor for proliferation of a bacterium of the genus *Faecalibacterium*;
(B') an improver for symptoms or conditions involving a bacterium of the genus *Faecalibacterium*; and
(C') an alternative to a saccharide that promotes proliferation of a bacterium of the genus *Faecalibacterium*.

The method and use of the present invention, and a bacterium of the genus *Acidipropionibacterium* or a processed product thereof can be put into practice by those skilled in the art in accordance with the contents of the specification with respect to a composition of the present invention.

According to an embodiment of the present invention, the following [1] to [15] are provided.

[1] A composition containing a bacterium of the genus *Acidipropionibacterium* or a processed product thereof and satisfying at least one requirement selected from the following (A) to (D):
(A) the composition is a composition for promoting proliferation of a bacterium of the genus *Faecalibacterium*;
(B) the composition is a composition for improving symptoms or conditions involving a bacterium of the genus *Faecalibacterium*;
(C) the composition is an alternative to a saccharide that promotes proliferation of a bacterium of the genus *Faecalibacterium*;
(D) a total content of a saccharide that promotes proliferation of a bacterium of the genus *Faecalibacterium* in the composition is 15 mass% or less.

[2] The composition according to [1], wherein the bacterium of the genus *Acidipropionibacterium* is *Acidipropionibacterium acidipropionici.*

[3] The composition according to [1] or [2], wherein the processed product is at least one selected from the group consisting of a suspension, a culture, culture supernatant, a fermented product, a heat-processed product, a ground material, a concentrate, a sterilized product, a liquefied product, a paste, a dried product and a diluted product of the bacterium of the genus *Acidipropionibacterium.*

[4] The composition according to any of [1] to [3], wherein the content of the bacterium of the genus *Acidipropionibacterium* or a processed product thereof is 0.01 mass% or more on a dry-weight basis.

[5] The composition according to any of [1] to [4], wherein the saccharide of (C) is at least one selected from a fermentable carbohydrate, a monosaccharide, a disaccharide and a polyol.

[6] The composition according to any of [1] to [5], wherein the saccharide of (C) is at least one selected from 1-kestose and inulin.

[7] The composition according to any of [1] to [6], wherein the total content of the saccharide of (C) is 0.01 to 15 mass%.

[8] The composition according to any of [1] to [7], wherein the bacterium of the genus *Faecalibacterium* is *Faecalibacterium prausnitzii.*

[9] The composition according to any of [1] to [8], wherein the composition is an oral composition.

[10] The composition according to any of [1] to [9], wherein the composition is a food composition or a pharmaceutical composition.

[11] The composition according to any of [1] to [10], for use as a probiotic, a prebiotic or a synbiotic.

[12] The composition according to any of [1] to [11], wherein the composition is a low-FODMAP diet or a low-FOLFAP diet.

[13] The composition according to any of [1] to [12], wherein the composition of (B) is a composition for improving at least one symptom or condition selected from the group consisting of irritable bowel syndrome (IBS), inflammatory bowel disease (IBD), glycometabolism, lipid metabolism, intestinal environment, immune status, oxidative stress, inflammation, colorectal cancer and depression.

[14] The composition according to any of [1] to [13], for a subject in need of a low-FODMAP diet or a low-FOLFAP diet.

[15] A composition containing a bacterium of the genus *Acidipropionibacterium* or a processed product thereof and at least one additive selected from the group consisting of a protein hydrolysate, a yeast extract, a meat extract and glucose.

### Examples

Now, the present invention will be more specifically described by way of Examples but the present invention is not limited by these Examples. Note that, unless otherwise specified, all ratios used in the present invention are on a mass basis. Unless otherwise specified, the units and measuring methods described in the specification are those according to the Japanese Industrial Standards (JIS).

### Bacterial species

The following bacterial strains were prepared.
(1) *F. prausnitzii* ATCC27768T
(2) *Bifidobacteirum longum* JCM1217T
(3) *A. acidipropionici* IFO12425
(4) *A. acidipropionici* P2002701
(5) *A. acidipropionici* P2002702
(6) *A. acidipropionici* DSM4900T
(7) *A. acidipropionici* P2002703

### Composition of medium

Compositions of agar medium 1, medium 2 and medium 3 used in the following experiments will be listed in Table 1 to Table 3 shown below.

**[Table 1]**

| Agar medium 1 | |
|---|---|
| Components | Amount Used |
| Encephalomyo infusion (solid) | 6.0 g |
| Animal tissue-peptone digested with pepsin | 6.0 g |
| Sodium chloride | 5.0 g |
| Glucose | 3.0 g |
| Gelatin hydrolysate | 14.5 g |
| Disodium phosphate | 2.5 g |
| Agar | 15.0 g |
| Yeast extract(Bacto (TM) Teast Etract) | 5.0 g |
| Hemin | 15.0 mg |
| L-cystein | 1.0 g |
| milliQ | 1000 mL |

**[Table 2]**

| Medium 2 | |
|---|---|
| Components | Amount Used |
| Encephalomyo infusion (solid) | 6.0 g |
| Animal tissue-peptone digested with pepsin | 6.0 g |
| Sodium chloride | 5.0 g |
| Glucose | 3.0 g |
| Gelatin hydrolysate | 14.5 g |
| Disodium phosphate | 2.5 g |
| Bacto (TM) Teast Etract | 5.0 g |
| Hemin | 15.0 mg |
| L-cystein | 1.0 g |
| milliQ | 1000 mL |

**[Table 3]**

| Medium 3 | |
|---|---|
| Components | Amount Used |
| Peptone | 10.0 g |
| Soybean peptone (soybean protein hydrolysate) | 3.0 g |
| Protease peptone | 10.0 g |
| Digested serum powder | 13.5 g |
| Yeast extract | 5.0 g |
| Meat extract | 2.2 g |
| Liver extract | 1.2 g |
| Glucose | 3.0 g |
| Potassium dihydrogen phosphate | 2.5 g |
| Sodium chloride | 3.0 g |
| Soluble starch | 5.0 g |
| L-cysteine hydrochloride | 0.3 g |
| Sodium thioglycolate | 0.3 g |
| milliQ | 1000 mL |

### Experimental Example 1: Examination of proliferation promoting effect of A. acidipropionici on F. prausnitzii in plate medium

### 1-1: Preparation of agar medium of test bacterial strain (F. prausnitzii ATCC27768T)

A test bacterial strain, *F. prausnitzii* ATCC27768T, was prepared as follows.

A frozen bacterial strain of *F. prausnitzii* ATCC27768T was suspended in medium 2 and then activated by culturing it at 37°C overnight under anaerobic conditions to obtain a culture solution. The culture solution was suspended in fresh medium 2 so as to obtain a final concentration of 1% and then cultured at 37°C overnight under anaerobic conditions. The culture solution (100 µL) thus obtained was smeared onto agar medium 1 to prepare an agar medium of the bacterial strain (*F. prausnitzii* ATCC27768T).

### 1-2: Preparation of control

A control was prepared using *Bifidobacteirum longum* JCM1217T as a test bacterial strain.

A frozen bacterial strain of *Bifidobacteirum longum* JCM1217T was suspended in medium 3 and then activated by culturing it at 37°C overnight under anaerobic conditions to obtain a culture solution. The culture solution was suspended in fresh medium 3 so as to obtain a final concentration of 1% and then cultured at 37°C overnight under anaerobic conditions to prepare a control.

### 1-3: Preparation of test sample

Test samples were prepared using 4 bacterial strains: *A*. *acidipropionici* P2002701, *A*. *acidipropionici* P2002702, *A*. *acidipropionici* DSM4900T and *A. acidipropionici* P2002703 as test bacterial strains as follows.

Each of the frozen bacterial strains of *A. acidipropionici* was suspended in medium 3 and then activated by culturing it at 30°C for 3 days under anaerobic conditions to obtain a culture solution. The culture solution was suspended in fresh medium 3 so as to obtain a final concentration of 1% and then cultured at 30°C for 3 days under anaerobic conditions. In this manner, test samples were prepared.

### 1-4: Examination of an effect of promoting proliferation of F. prausnitzii

To the agar medium of test bacterial strain (*F. prausnitzii* ATCC27768T), a control or a test sample was added dropwise and subjected to stationary culture to confirm the presence or absence of a proliferation promoting effect on *F. prausnitzii* ATCC27768T, in accordance with the scheme shown in Figure 1. More specifically, to the agar medium of the test bacterial strain, 1 µL of the culture solution of a control or a test sample was added dropwise. The plate medium was subjected to stationary culture performed at 37°C for 4 days under anaerobic conditions.

The results are shown in Table 4. In Table 4, a case where promotion of proliferation of *F. prausnitzii* around the test bacterial strain was confirmed was evaluated as "+", whereas a case where promotion of proliferation of *F. prausnitzii* around the test bacterial strain was not confirmed was evaluated as "-", as shown in Figure 1. *A. acidipropionici* P2002702 was added dropwise, subjected to stationary culture and photographed. The photograph is shown in Figure 2. In Figure 2, the black dot, which was observed in the upper portion of the petri dish, corresponds to a colony of *A. acidipropionici* P2002702. In Figure 2, proliferation of *F*. *prausnitzii* was observed around the black dot. The area in which *F. prausnitzii* proliferated is twice as large as that of the dot.

**[Table 4]**

| Test bacterial strain or medium | | Evaluation |
|---|---|---|
| Medium 3 | | - |
| *Bifidobacteirum longum* | JCM 1217T | - |
| *A. acidipropionici* | P2002701 | + |
| | P2002702 | + |
| | DSM4900T | + |
| | P2002703 | + |

### Experimental Example 2: Examination of proliferation promoting effect of A. acidipropionici on F. prausnitzii in liquid medium

### 2-1: Preparation of culture solution (liquid medium) of test bacterial strain (F. prausnitzii ATCC27768T)

A liquid solution (liquid medium) of a test bacterial strain, *F*. *prausnitzii* ATCC27768T, was prepared as follows.

A frozen bacterial strain of *F. prausnitzii* ATCC27768T was suspended in medium 2 and then activated by culturing it at 37°C overnight under anaerobic conditions to obtain a culture solution. The culture solution was suspended in fresh medium 2 so as to obtain a final concentration of 1% and cultured at 37°C overnight under anaerobic conditions. The culture solution obtained was further suspended in fresh medium 2 so as to obtain a final concentration of 1% to prepare a culture solution of the bacterial strain (*F. prausnitzii* ATCC27768T).

### 2-2: Preparation of test composition

Test samples were prepared using 4 bacterial strains: *A*. *acidipropionici* P2002701, *A*. *acidipropionici* P2002702, *A*. *acidipropionici* DSM4900T and *A. acidipropionici* P2002703 as test bacterial strains, as follows.

Each of the frozen bacterial strains of *A. acidipropionici* was suspended in medium 3 and then activated by culturing it at 30°C for 3 days under anaerobic conditions to obtain a culture solution. The culture solution was suspended in fresh medium 3 so as to obtain a final concentration of 1% and cultured at 30°C for 3 days under anaerobic conditions. Each of the culture solutions obtained was centrifuged at 8,000 × g at 4°C for 10 minutes and the bacterial cell pellet was washed twice with saline. Thereafter, the bacterial cell pellet was suspended in saline so as to obtain OD₆₀₀ = 1.0 to prepare a test sample.

### 2-3: Examination of effect of promoting proliferation of F. prausnitzii

To the culture solution (liquid medium) of a test bacterial strain, a test sample was added dropwise and co-cultured for 24 hours. From the culture solution obtained, DNA was extracted and subjected to qPCR, and then, a test for confirming whether or not *A. acidipropionici* increases the bacterial count of *F. prausnitzii* was carried out. The confirmation test of each bacterial species was repeated three times.

More specifically, to the culture solution (liquid medium) of a test bacterial strain, a test sample or a control (saline) was added so as to obtain a final concentration of 1%. After co-culturing for 24 hours, DNA was extracted from the culture solution and subjected to qPCR.

Figure 3 shows the bacterial counts of *F. prausnitzii* after co-culturing.

As shown in Figure 3, in all of the culture systems in which *A. acidipropionici* was co-cultured, the bacterial count of F. *prausnitzii* significantly increased, compared to the control (saline). From the results, it was confirmed that *A. acidipropionici* promotes proliferation of *F. prausnitzii.*

### Experimental Example 3: Examination of proliferation promoting effect of culture supernatant of A. acidipropionici on F. prausnitzii

### 3-1: Preparation of culture solution of test bacterial strain F. prausnitzii ATCC27768T)

A culture solution of a test bacterial strain, *F. prausnitzii* ATCC27768T was prepared as follows.

A frozen bacterial strain of *F. prausnitzii* ATCC27768T was suspended in medium 2 and then activated by culturing it at 37°C overnight under anaerobic conditions to obtain a culture solution. The culture solution was suspended in fresh medium 2 so as to obtain a final concentration of 1% and cultured at 37°C overnight under anaerobic conditions. The culture solution obtained was further suspended in fresh medium 2 so as to obtain a final concentration of 1% to prepare a culture solution of the bacterial strain (*F. prausnitzii* ATCC27768T).

### 3-2: Preparation of test sample

Test samples as culture supernatants were prepared using 3 bacterial strains: *A. acidipropionici* IFO12425, *A. acidipropionici* P2002701, *A. acidipropionici* P2002702 as test bacterial strains, as follows.

Each of the frozen bacterial strains of *A. acidipropionici* was suspended in medium 3 and then activated by culturing it at 30°C for 3 days under anaerobic conditions. Each of the culture solutions obtained was suspended in fresh medium 3 so as to obtain a final concentration of 1% and cultured at 30°C for 5 days under anaerobic conditions. The culture solution obtained was centrifuged at 8,000 × g at 4°C for 10 minutes and the supernatant was collected. Thereafter, the supernatant obtained was sterilized through filtration by a 0.22 µm-filter to completely remove bacterial cells. In this manner, test samples were prepared.

Note that, medium 3 was sterilized through filtration by a 0.22 µm-filter and used as a control.

### 3-3: Examination of proliferation promoting effect on F. prausnitzii

To the culture solution of a test bacterial strain, a control or culture supernatant of a test sample was added and the mixed solution was cultured for 20 hours. From the culture solution obtained, DNA was extracted and subjected to qPCR, and then, a test for confirming whether or not the culture supernatant of *A. acidipropionici* increases the bacterial count of *F. prausnitzii,* was carried out. The confirmation test of each bacterial species was repeated three times.

More specifically, to the culture solution of a test bacterial strain, a test sample or a control was added so as to be 10% (v/v). After the culture solution was cultured for 20 hours, DNA was extracted from the culture solution and subjected to qPCR.

Figure 4 shows the bacterial counts of *F. prausnitzii* when culture supernatants were added. As shown in Figure 4, in the culture systems in which culture supernatants (test samples) of *A. acidipropionici* were added, the bacterial counts of *F. prausnitzii* significantly increased, compared to the case where a control was used.

Accordingly, it was confirmed that the culture supernatant (test sample) of *A. acidipropionici* promotes proliferation of *F. prausnitzii.*

## Claims

1. A composition comprising a bacterium of the genus *Acidipropionibacterium* or a processed product thereof and satisfying at least one requirement selected from the following (A) to (D):
(A) the composition is a composition for promoting proliferation of a bacterium of the genus *Faecalibacterium*;
(B) the composition is a composition for improving symptoms or conditions involving a bacterium of the genus *Faecalibacterium*;
(C) the composition is an alternative to a saccharide that promotes proliferation of a bacterium of the genus *Faecalibacterium*;
(D) a total content of a saccharide that promotes proliferation of a bacterium of the genus *Faecalibacterium* in the composition is 15 mass% or less.

2. The composition according to claim 1, wherein the bacterium of the genus *Acidipropionibacterium* is *Acidipropionibacterium acidiorooionici.*

3. The composition according to claim 1 or 2, wherein the processed product is at least one selected from the group consisting of a suspension, a culture, culture supernatant, a fermented product, a heat-processed product, a ground material, a concentrate, a sterilized product, a liquefied product, a paste, a dried product and a diluted product of the bacterium of the genus *Acidipropionibacterium.*

4. The composition according to any one of claims 1 to 3, wherein the content of the bacterium of the genus *Acidipropionibacterium* or a processed product thereof is 0.01 mass% or more on a dry-weight basis.

5. The composition according to any one of claims 1 to 4, wherein the saccharide of (C) is at least one selected from a fermentable carbohydrate, a monosaccharide, a disaccharide and a polyol.

6. The composition according to any one of claims 1 to 5, wherein the saccharide of (C) is at least one selected from 1-kestose and inulin.

7. The composition according to any one of claims 1 to 6, wherein the total content of the saccharide of (C) is 0.01 to 15 mass%.

8. The composition according to any one of claims 1 to 7, wherein the bacterium of the genus *Faecalibacterium* is *Faecalibacterium prausnitzii.*

9. The composition according to any one of claims 1 to 8, wherein the composition is an oral composition.

10. The composition according to any one of claims 1 to 9, wherein the composition is a food composition or a pharmaceutical composition.

11. The composition according to any one of claims 1 to 10, for use as a probiotic, a prebiotic or a synbiotic.

12. The composition according to any one of claims 1 to 11, wherein the composition is a low-FODMAP diet or low-FOLFAP diet.

13. The composition according to any one of claims 1 to 12, wherein the composition of (B) is a composition for improving at least one symptom or condition selected from the group consisting of irritable bowel syndrome (IBS), inflammatory bowel disease (IBD), glycometabolism, lipid metabolism, intestinal environment, immune status, oxidative stress, inflammation, colorectal cancer and depression.

14. The composition according to any one of claims 1 to 13, for a subject in need of a low-FODMAP diet or a low-FOLFAP diet.

15. A composition comprising:
a bacterium of the genus *Acidipropionibacterium* or a processed product thereof; and
at least one additive selected from the group consisting of a protein hydrolysate, a yeast extract, a meat extract and glucose.
